# EUROPEAN PATENT APPLICATION

(11) **EP 4 539 486 A1**
(43) Date of publication of application: **16.04.2025**
(21) Application number: 24187218.3
(22) Date of filing: 08.07.2024
(51) Int. Cl.: H04N 23/63, H04N 23/51, H04N 23/55, H04N 23/56, H04N 23/667, H04N 23/71, H04N 23/74, A61F 9/06, B23K 9/32, F16P 1/06

(54) **CAMERA UNIT AND WELDING INFORMATION PROVIDING APPARATUS COMPRISING THE SAME**

(30) Priority: 10.10.2023 KR 20230133964
(71) Applicant: Otos Wing Co., Ltd., Seoul 08521 (KR)
(72) Inventor: HUH, Moon Young, 08521 Seoul (KR); HUH, Sung Won, 06084 Seoul (KR)
(74) Representative: HGF

(57) **Abstract**

Provided are a camera unit and a welding information providing apparatus including the same. The camera unit may include a camera module configured to obtain a welding image frame in a welding operation area according to photographing conditions, an illuminance sensor configured to detect an intensity of light within the welding operation area, and a controller configured to determine, based on the intensity of light detected by the illuminance sensor, an operation mode of the camera module to be one of a first mode and a second mode, and change photographing conditions of the camera module to preset photographing setting values according to the determined mode.

## Description

### BACKGROUND

### 1. Field

The present disclosure relates to a camera unit that changes photographing conditions based on the intensity of light and a welding information providing apparatus including the camera unit.

### 2. Description of the Related Art

An operator wears a protector for protection from light and high temperature heat generated during a welding process. While the operator wears the protector, the operator may only check processing of the welding through the protector, and thus, the operator has to remove the protector and check with the naked eye in order to identify various information for a welding operation, such as conditions set in a welding apparatus.

When a skill level of an operator is not high, especially when an automatic welding mask and a manual welding mask are worn, the operator may see only a portion adjacent to welding light, and it is difficult for the operator to recognize a specific welding situation such as the surroundings of the welding. Accordingly, high-definition images are provided to operators so that they can visually check the environment around a welding site.

However, during welding, because the illuminance/brightness of the welding light spot is very high, the image capturing environment when welding is in progress greatly differs from the image capturing environment when welding is temporarily stopped.

Therefore, there is a need for technology for obtaining and providing accurate images of a welding operation by adaptively changing the photographing conditions of a camera according to the image capturing environment.

### SUMMARY

An embodiment of the present disclosure provides a camera unit and a welding information providing apparatus including the camera unit, wherein the camera unit is configured to adaptively change an operation mode of a camera module and photographing conditions according to the operation mode according to an image capturing environment to obtain and provide a clear image of a welding operation so as to allow the operator to accurately perform a welding process.

An embodiment of the present disclosure provides a welding information providing apparatus configured to determine an operation mode of a camera unit to be one of a first mode and a second mode according to intensity of light in a welding operation area, detected by an illuminance sensor, and change photographing conditions of the camera unit to photographing setting values preset in a memory in accordance with the determine mode, so as to quickly change the photographing conditions of the camera unit without delay.

In addition, an embodiment of the present disclosure provides a welding information providing apparatus for obtaining a welding image in which a certain amount of welding light is shielded, without separately processing a captured image, by immediately controlling, through a processor separately provided in a light blocking panel, the light blocking degree of the light blocking panel according to intensity of light in a welding operation area, detected by an illuminance sensor.

The objectives to be solved by the present disclosure are not limited to those stated above, and other objectives and advantages of the present disclosure that are not mentioned are understood through the following description and can be understood more clearly through the examples of the present disclosure. In addition, the objectives and advantages to be solved by the present disclosure can be realized by the means and combinations thereof indicated in the claims.

Additional aspects will be set forth in part in the description which follows and, in part, will be apparent from the description, or may be learned by practice of the presented embodiments of the disclosure.

A camera unit according to an embodiment of the present disclosure includes a camera module configured to obtain a welding image frame in a welding operation area according to photographing conditions, an illuminance sensor configured to detect an intensity of light within the welding operation area, and a controller configured to determine, based on the intensity of light detected by the illuminance sensor, an operation mode of the camera module to be one of a first mode and a second mode, and change photographing conditions of the camera module to preset photographing setting values according to the determined mode.

The camera module may include an image sensor that recognizes light from a welding operation, and the camera unit may further include a light blocking panel disposed in front of the camera module including the image sensor and configured to block a portion of light incident on the welding operation and transmit the remaining light to the image sensor.

The light blocking panel may include a processor electrically connected to the illuminance sensor, and the processor may be configured to control a light blocking degree of the light blocking panel based on the intensity of the light.

The processor may be further configured to control the light blocking degree of the light blocking panel in proportion to the intensity of the light, or control the light blocking degree of the light blocking panel to be a preset value based on a comparison result between the intensity of the light and a preset intensity.

The camera unit may further include a lighting unit that irradiates illumination light, wherein the controller adjusts an output intensity of the illumination light emitted from the lighting unit, based on the intensity of the light.

The illuminance sensor may detect the intensity of the light at intervals of a set cycle, and the controller may count the number of times the operation mode of the camera module changes according to a change in the intensity of the light, and generate and output a notification message when the counted number exceeds a preset number during a preset time.

A welding information providing apparatus according to one embodiment of the present disclosure includes a main body provided to be worn by a user, a camera unit disposed outside the main body and configured to obtain a welding image frame in a welding operation area according to photographing conditions, an illuminance sensor configured to detect an intensity of light within the welding operation area, and a processor configured to determine, based on an intensity of light detected by the illuminance sensor, an operation mode of the camera unit to be one of a first mode and a second mode, and change photographing conditions of the camera unit to preset photographing setting values according to the determined mode.

In addition, another method for implementing the present disclosure, another system, and a computer-readable recording medium storing a computer program for executing the method may be further provided.

In addition to the aforesaid details, other aspects, features, and advantages will be clarified from the following drawings, claims, and detailed description.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a diagram for describing a structure of a welding system according to an embodiment of the present disclosure;
FIG. 2 is a diagram illustrating an example of a configuration of a welding information providing apparatus according to an embodiment of the present disclosure;
FIG. 3 is a diagram illustrating another example of a configuration of a welding information providing apparatus according to an embodiment of the present disclosure;
FIG. 4 is a diagram illustrating another example of a configuration of a welding information providing apparatus according to another embodiment of the present disclosure;
FIG. 5 is a perspective view of a welding information providing apparatus according to an embodiment of the present disclosure;
FIG. 6 is a cross-sectional view for describing inside of the welding information providing apparatus of FIG. 5;
FIG. 7 is a diagram only showing elements of a camera unit of FIG. 6;
FIG. 8 is a diagram illustrating a camera unit according to another embodiment;
FIG. 9 is a block diagram for describing a method of controlling a light blocking panel within a camera unit;
FIG. 10 is a diagram illustrating an example of photographing setting values for each mode stored in a memory of FIG. 2; and
FIG. 11 is a flowchart for describing an operation method of a welding information providing apparatus, according to this embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

The advantages and features of the present disclosure and methods for achieving them will become clear by referring to the embodiments described in detail together with the accompanying drawings. However, the present disclosure is not limited to the embodiments presented below, but may be implemented in various different forms, and should be understood to include all conversions, equivalents, and substitutes included in the spirit and technical scope of the present disclosure. The embodiments presented below are provided to ensure that the present disclosure is complete and to fully inform those skilled in the art of the scope of the present disclosure. In describing the present disclosure, if it is determined that a detailed description of related known technologies may obscure the gist of the present disclosure, the detailed description will be omitted.

The terms used in this application are only used to describe specific embodiments and are not intended to limit the present disclosure. An expression used in the singular encompasses the expression of the plural, unless it has a clearly different meaning in the context. Also, it should be understood that the terms "comprises", "has", etc. when used in the present application, specify the presence of stated features, integers, steps, operations, elements, and/or components, but do not preclude the presence or addition of one or more other features, integers, steps, operations, elements, components, and/or groups thereof. While such terms as "first," "second," etc., may be used to describe various components, such components must not be limited to the above terms. The above terms are used only for the purpose of distinguishing one component from another.

Additionally, in this application, a "unit" may be a hardware component, such as a processor or circuit, and/or a software component executed by the hardware component, such as a processor.

Hereinafter, embodiments according to the present disclosure will be described in detail with reference to the accompanying drawings. In the description with reference to the accompanying drawings, identical or corresponding components are assigned the same drawing numbers and duplicate descriptions thereof are omitted.

While such terms as "first," "second," etc., may be used to describe various components, such components must not be limited to the above terms. The above terms are used only to distinguish one component from another.

An expression used in the singular encompasses the expression of the plural, unless it has a clearly different meaning in the context.

In the present specification, it is to be understood that the terms such as "including," "having," and "comprising" are intended to indicate the existence of the features or components disclosed in the specification, and are not intended to preclude the possibility that one or more other features or components may exist or may be added.

In cases where an embodiment can be implemented differently, a specific process sequence may be performed differently from the described sequence. For example, two processes described in succession may be performed substantially at the same time, or may be performed in an order opposite to that in which they are described.

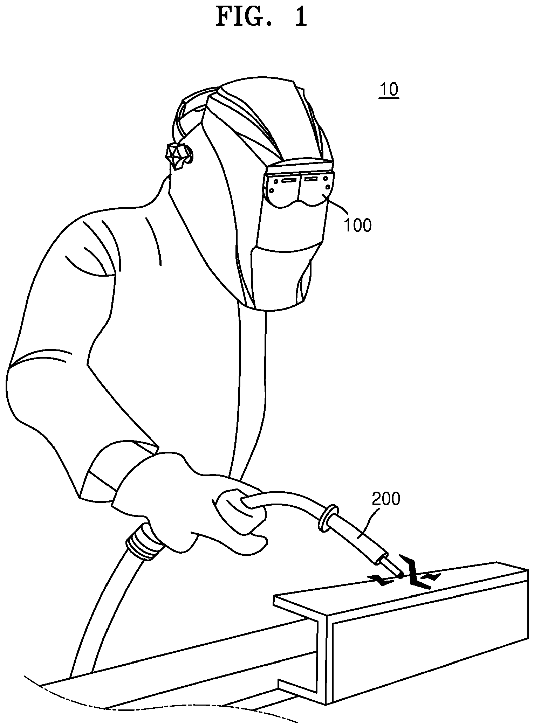

FIG. 1 is a diagram for describing a structure of a welding system according to an embodiment of the present disclosure.

Referring to FIG. 1, a welding system 10 according to the present disclosure may include a welding information providing apparatus 100 and a welding torch 200. The welding information providing apparatus 100 and the welding torch 200 may be connected to each other through a communication network to transmit and receive data. The welding information providing apparatus 100 and the welding torch 200 may be operated while matching in one-to-one correspondence, but one or more embodiments are not limited thereto, and a one-to-n relationship is possible. That is, n welding torches 200 may be connected to one welding information providing apparatus, or one welding torch 200 may be connected to n welding information providing apparatuses 100. In addition, the welding information providing apparatus 100 and the welding torch 200 may exchange data by communicating with an additional server (not shown).

The welding information providing apparatus 100 may provide information about a welding situation to an operator. In detail, the welding information providing apparatus 100 may obtain a welding image that is obtained by using at least one camera mounted on the welding information providing apparatus 100, and generate a composite image based on the welding image and display the image. Here, the welding information providing apparatus 100 may generate the composite image by using a high dynamic range (HDR) technology, and may display a high-definition composite image to the operator. Here, the operator may visually check information about a shape of welding beads and a surrounding environment other than a portion adjacent to the welding light through the high-definition composite image.

The welding information providing apparatus 100 according to an embodiment of the present disclosure may obtain images from two or more cameras and display the respective images through at least one display in order to provide high-definition composite welding image. Here, the welding information providing apparatus 100 may synthesize images by repeatedly capturing images while varying a shutter speed, an ISO sensitivity, and a gain value of each camera. The welding information providing apparatus 100 according to an embodiment of the present disclosure may improve the image quality through a contrast ratio treatment on the obtained composite image.

Also, the welding information providing apparatus 100 of the present disclosure may provide a function of displaying welding information in preferred color (e.g., green and blue) by using RGB. In addition, the welding information providing apparatus 100 of the present disclosure may provide a function of correcting power of a magnifying glass (e.g., screen enlargement and reduction). Also, the welding information providing apparatus 100 of the present disclosure may provide a temperature composite image by using an additional thermal imaging camera. Here, the welding information providing apparatus 100 may indicate a welding temperature in a color. The welding information providing apparatus 100 of the present disclosure may support a function of providing sound (e.g., notification alarm) or guidance voice with respect to the above-described functions.

The welding torch 200 according to an embodiment of the present disclosure may sense a welding situation including a welding temperature, a welding direction, a welding inclination, a welding speed, a distance between a base material and the welding torch, etc. of a real-time welding operation, through at least one sensor. The welding torch 200 may monitor a state of the torch and may change a setting value of a welding torch operation according to the welding situation.

The welding information providing apparatus 100 of the present disclosure may receive information about an operation setting and an operation state from the welding torch 200 through a communication network connected to the welding torch 200, and may provide the operator with operation information based on the received welding information through visual feedback.

For example, when receiving sensing information about the welding temperature value, the welding information providing apparatus 100 may output a notification corresponding to the temperature value in various methods, e.g., light, vibration, message, etc. Here, the notification may be visual feedback provided on a display unit or a display, or may be audible feedback provided through sound (e.g., notification alarm) or guiding voice.

In addition, the sensing information about the temperature value may include information about whether the temperature value exceeds a temperature range set in advance, etc. Also, the sensing information about the temperature value may include a numerical value, a grade, a level, etc. corresponding to the temperature value on a welding surface.

When it is determined that the temperature values of the torch and the welding surface exceed the temperature range set in advance, the welding information providing apparatus 100 according to an embodiment of the present disclosure may guide the operator to stop the operation. The welding performed at the temperature exceeding the temperature range set in advance has a risk of quality degradation, and the operator may be guided to adjust the temperature value of the torch.

When detecting that a current or voltage status of the welding torch 200 is abnormal, the welding information providing apparatus 100 according to an embodiment of the present disclosure may provide the visual feedback for warning.

Here, the visual feedback may denote providing of an icon indicating danger on a part of the display unit of the welding information providing apparatus 100, which is displaying the working site. In another example, the welding information providing apparatus 100 may provide an operation suspending guidance through the visual feedback by repeatedly increasing and decreasing a saturation of a certain color (e.g., red) on the entire screen of the display unit.

In addition, when the number of times that the operation mode of the camera unit (or the photographing condition of the camera unit) changes according to a change in light intensity exceeds a preset number during a preset time, the welding information providing apparatus 100 according to an embodiment of the present disclosure may generate and output a notification message to limit the progress of frequent welding operation within a short period of time so that eye fatigue may be reduced.

According to an embodiment of the present disclosure, the welding information providing apparatus 100 may sense the welding information via a sensor (e.g., first sensor) included in the welding information providing apparatus 100, in addition to at least one sensor (e.g., second sensor) included in the welding torch 200. Here, the welding information including the welding situation, e.g., a degree of light related to the real-time welding operation, a welding temperature, a welding direction, a welding inclination, a welding speed, a distance between the base material and the welding torch, etc. may be sensed via at least one sensor.

Likewise, the welding information providing apparatus 100 may provide guidance corresponding to the welding information based on the welding information sensed by the sensor (e.g., first sensor) included in the welding information providing apparatus 100.

According to an embodiment of the present disclosure, the welding information providing apparatus 100 may change a movement of the welding torch by sensing a preset user movement or a preset user voice after an operation suspension guidance is provided.

In another embodiment, when communication between the welding information providing apparatus 100 and the welding torch 200 is not sufficiently performed, the welding information providing apparatus 100 may obtain temperature values of the torch and the welding surface through an image sensing provided therein. For example, the welding information providing apparatus 100 may obtain temperature values of the torch and the welding surface based on image data obtained through a thermal imaging camera.

Although the above example illustrates that information received from the welding torch 200 only includes welding temperature information, the welding information providing apparatus 100 may provide various guidance for various welding information.

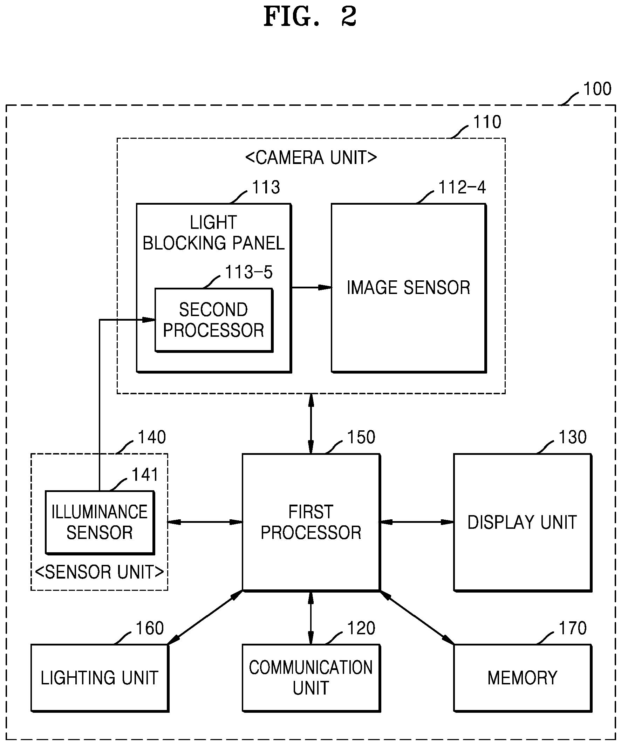

FIG. 2 is a diagram illustrating an example of a configuration of a welding information providing apparatus according to an embodiment of the present disclosure.

Referring to FIG. 2, the welding information providing apparatus 100 may include a camera unit 110, a communication unit 120, a display unit 130, a sensor unit 140, a first processor 150, a lighting unit 160, and a memory 170.

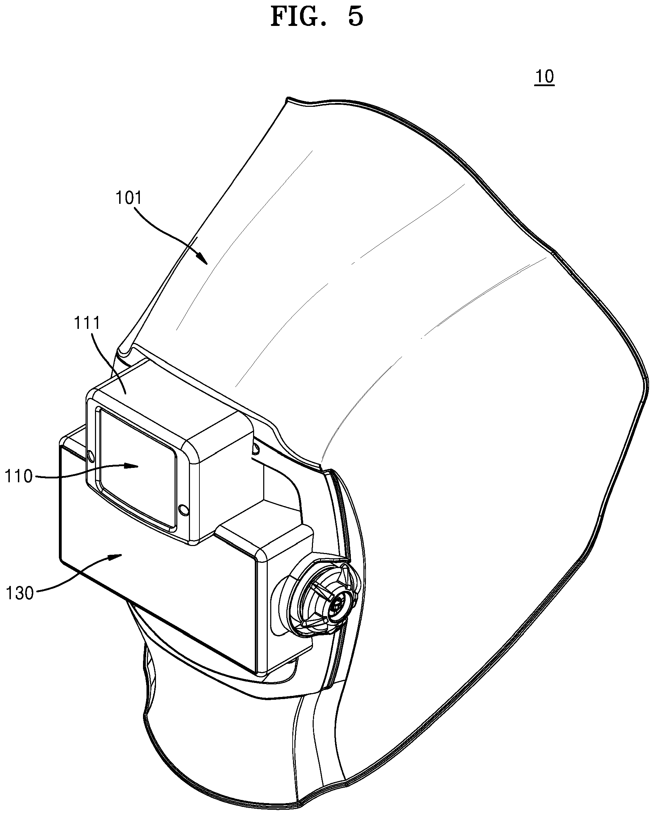

The camera unit 110 may include at least one camera, and may include a camera for capturing images of a welding operation site. The camera unit 110 according to an embodiment of the present disclosure may be a camera located adjacent to the display unit 130 of the welding information providing apparatus 100. For example, a first camera and a second camera of the camera unit 110 may be symmetrically mounted on one area of a front portion of the welding information providing apparatus 100, respectively. In another embodiment, the camera unit 110 may be formed to be attachable and detachable, and may be mounted while changing its position as necessary. As described above, the camera unit 110 may be mounted to be adjacent to the display unit 130, but may be mounted by changing the position thereof to a side portion of a main body (101 of FIG. 5) as necessary. Alternatively, the camera unit 110 may be mounted on the upper portion of the main body 101, that is, on the head of the user.

The camera unit 110 (specifically, a camera module 112 within the camera unit 110) may obtain welding image frames in a welding operation area according to photographing conditions. At this time, the camera unit 110 (i.e., the camera module 112) may operate in a plurality of operation modes with different photographing conditions (for example, a 'welding mode' in which welding operation is performed, a normal mode' in which no welding operation is in progress) under the control of the first processor 150. The camera unit 110 may receive a control command from the first processor 150 and change, in response to the control command, settings such as shutter speed, ISO sensitivity, and gain and capture an image of a welding operation site, according to the operation mode determined by the first processor 150. The camera unit 110 may include the first camera and the second camera which may capture images of the welding operation site under different photographing settings, respectively.

In addition, the camera unit 110 may include an image sensor 112-4 that recognizes light from a welding operation and generates an image signal that is a basis of a welding image frame, and a light blocking panel 113 which is arranged in front of the image sensor 112-4 (e.g. in front of the camera module 112 including the image sensor 112-4) and able to perform a light blocking function. The light blocking panel 113 may block a portion of light incident on the welding operation and transmit the remaining light to the image sensor 112-4.

The light blocking panel 113 may include a second processor 113-5 that is electrically connected to an illuminance sensor 141 of the sensor unit 140. The second processor 113-5 may control the light blocking degree of the light blocking panel 113 based on the intensity of light detected by the illuminance sensor 141. In an embodiment, the second processor 113-5 may control the degree of light blocking of the light blocking panel 113 in proportion to the intensity of light, or control the light blocking degree of the light blocking panel 113 to be a preset value, based on a comparison result between the intensity of light and a preset intensity. For example, the second processor 113-5 may increase the light blocking degree of the light blocking panel 113 as the intensity of light increases. In addition, when the intensity of light exceeds a first intensity, the second processor 113-5 may control the light blocking degree of the light blocking panel 113 to be a first value, and when the intensity of light is equal to or less than the first intensity and exceeds a second intensity, the second processor 113-5 may control the light blocking degree of the light blocking panel 113 to be a second value lower than the first value. Additionally, when the intensity of light is equal to or less than the second intensity, the second processor 113-5 may control the light blocking degree of the light blocking panel 113 to be a third value lower than the second value. Here, the second intensity may be lower than the first intensity, but is not limited thereto and may be the same as the first intensity.

In another embodiment, the second processor 113-5 may control the degree of light blocking of the light blocking panel 113 based on the operation mode of the camera unit 110. When the operation mode of the camera unit 110 is determined to be a first mode by the first processor 150, the second processor 113-5 may control the degree of light blocking of the light blocking panel 113 to be a preset first value to correspond to the first mode. When the operation mode of the camera unit 110 is determined to be a second mode by the first processor 150, the second processor 113-5 may control the degree of light blocking of the light blocking panel 113 to be a preset second value to correspond to the second mode.

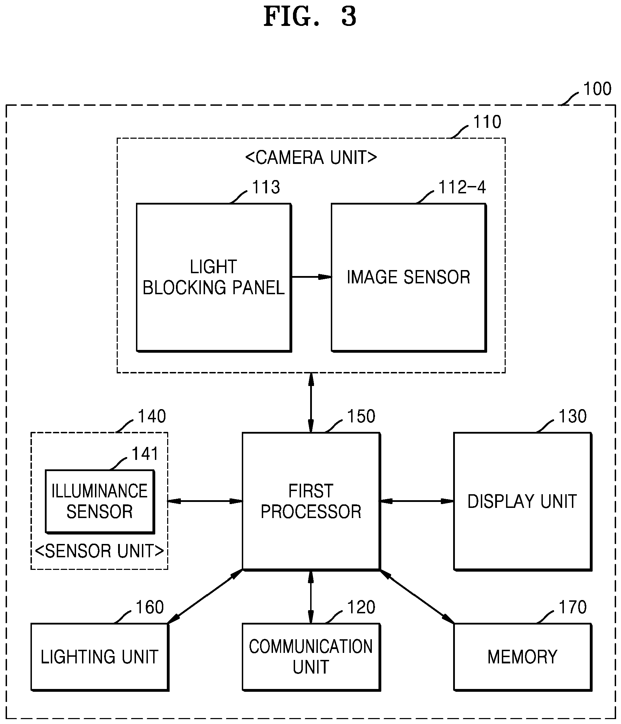

The light blocking panel 113 may be controlled by the second processor 113-5 connected to the illuminance sensor 141, but is not limited thereto, and may also be controlled by the first processor 150 as shown in FIG. 3. The first processor 150 may control the light blocking degree of the light blocking panel 113 based on the intensity of light detected by the illuminance sensor 141, or control the light blocking degree of the light blocking panel 113 based on a mode determined based on the light intensity.

The camera unit 110 according to an embodiment of the present disclosure may include a thermal imaging camera. The welding information providing apparatus 100 may obtain a temperature image by synthesizing a thermal image obtained by the thermal imaging camera with an image of a welding site.

According to an embodiment of the present disclosure, the welding information providing apparatus 100 may further include the lighting unit 160 electrically connected to the first processor 150. Here, the lighting unit 160 may be located outside the camera unit 110, but is not limited thereto and may be located inside the camera unit 110.

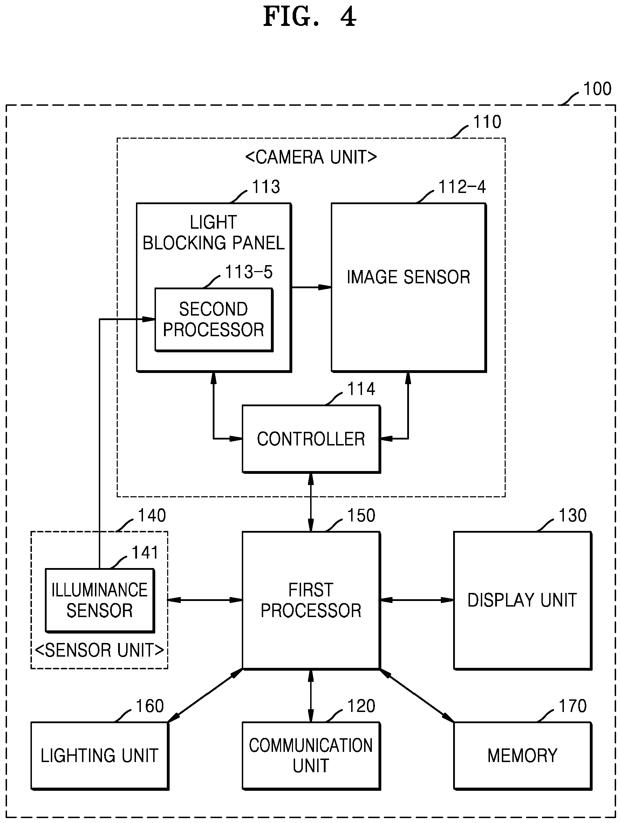

The lighting unit 160 is located outside the welding information providing apparatus 100 and is configured to irradiate illumination light toward at least a welding operation area. The lighting unit 160 may include a plurality of LED modules, and the output intensity (degree) of the illumination light emitted through the lighting unit 160 may be adjusted according to the control by the first processor 150 (or the controller 114 of FIG. 4) based on the intensity of light detected by the illuminance sensor 141. The first processor 150 may increase the output intensity of the illumination light emitted from the lighting unit 160 as the intensity of the light detected by the illuminance sensor 141 decreases.

According to an embodiment, the lighting unit 160 may operate in conjunction with the operation of the camera unit 110 under the control of the first processor 150 (or the controller 114 of FIG. 4). For example, when the operation mode of the camera unit 110 is determined to be the first mode, the first processor 150 may change the output intensity of illumination light emitted from the lighting unit 160, based on a first output value preset in the memory 170 to correspond to the first mode, and when the operation mode of the camera unit 110 is determined to be the second mode, the first processor 150 may change the output intensity of the illumination light emitted from the lighting unit 160, based on a second output value preset in the memory 170 to correspond to the second mode.

The communication unit 120 is a component for receiving welding information from the welding torch 200 and transmitting a command for controlling the welding torch 200. According to an embodiment of the present disclosure, the communication unit 120 may transmit a synthesized image to an external device in addition to the welding torch 200. Here, the external device may include various devices including a communication module, such as a smart phone of an operator/third party, a computer, etc.

The communication unit 120 may be a component that performs communication with various types of external devices according to various types of communication methods. The communication unit 120 may include at least one of a Wi-Fi chip, a Bluetooth chip, a wireless communication chip, and a near-field communication (NFC) chip. In particular, when a Wi-Fi chip or a Bluetooth chip is used, various connection information such as a service set identifier (SSID), a session key, etc. is transmitted/received first, and then, communication is connected using the above connection information and various information may be transmitted/received. The wireless communication chip refers to a chip that performs communication according to various communication standards such as IEEE, Zigbee, 3rd Generation (3G), 3rd Generation Partnership Project (3GPP), Long Term Evolution (LTE), etc. The NFC chip refers to a chip operating in an NFC type using a frequency band of 13.56 MHz from among various RF-ID frequency bands such as 135 kHz, 13.56 MHz, 433 MHz, 860 to 960 MHz, 2.45 GHz, etc.

The display unit 130 is a component for providing a high-definition synthesized image to the operator. In detail, the display unit 130 may be implemented in the form of goggle glasses including a display for displaying, to the operator, a synthesized image obtained by synthesizing images obtained via the camera unit 110.

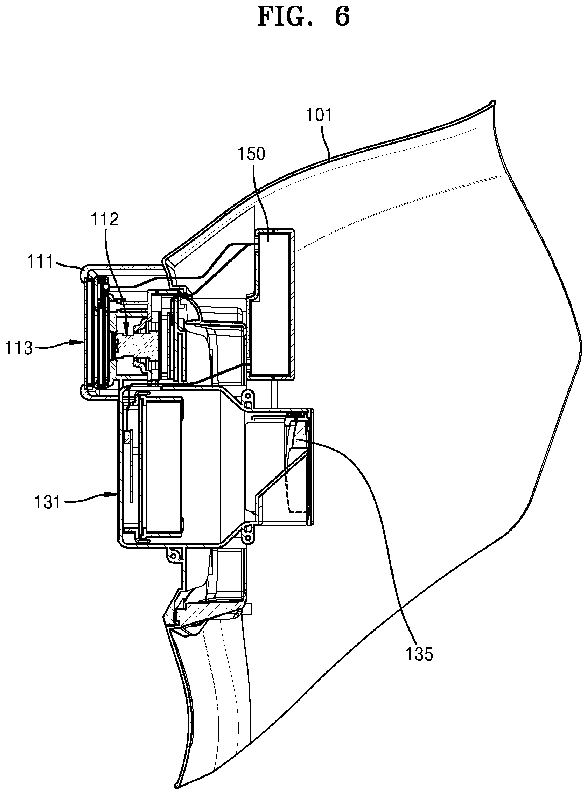

According to an embodiment of the present disclosure, the rear portion of the display unit 130, that is, the portion facing the user, may include the display 131 (refer to FIG. 6) for displaying a high-definition image to the user and a main lens member 135 (see FIG. 6) for seeing the display 131.

The display included in the display unit 130 may display a high-definition composite image so that the operator may visually check the surrounding environment (e.g., a shape of a previously worked welding bead, etc.) other than a portion adjacent to the welding light. In addition, the display unit 130 may guide the operator with visual feedback (e.g., a welding progress direction) on a welding progress state.

The display 131 included in the display unit 130 may be implemented as various display techniques such as a liquid crystal display (LCD), an organic light emitting diode (OLED), light-emitting diode (LED), liquid crystal on silicon (LcoS), or digital light processing (DLP), etc. Here, the display according to an embodiment of the present disclosure is implemented as a panel of an opaque material and the operator may not be directly exposed to harmful light. However, one or more embodiments are not limited thereto, and the display may be implemented as a transparent display.

The sensor unit 140 may include a plurality of sensor modules configured to detect various types of information regarding a welding site and acquire welding information. Here, the welding information may include a welding temperature, a welding direction, a welding inclination, a welding speed, a distance between the base material and the welding torch from the real-time welding operation. Moreover, the sensor unit 140 may include an optical sensor that is formed to detect a degree of light at least in the welding operation area.

According to an embodiment of the present disclosure, the sensor unit 140 may include the illuminance sensor 141 that detects the intensity of light within the welding operation area. The illuminance sensor 141 may obtain information about the intensity of light (e.g., welding light intensity, ambient light intensity) at the welding site. The illuminance sensor 141 may be included in the sensor unit 140, but is not limited thereto and may be included in, for example, the camera unit 110. In addition to the illuminance sensor, the sensor unit 140 may further include various types of sensors such as a proximity sensor, a noise sensor, a video sensor, an ultrasonic sensor, and an RF sensor, and may detect various changes related to a welding operation environment.

The first processor 150 may synthesize welding image frames received via the camera unit 110 to generate a high-definition synthesized image. The first processor 1150 may obtain the composite image by synthesizing in parallel the welding image frames that are obtained by the camera unit 110 in a time-serial order by setting different photographing conditions for each frame. In detail, the first processor 150 may control the camera unit 110 to capture images by changing the ISO sensitivity, gain value, the shutter speed, etc. of the camera unit 110.

Here, the first processor 150 may differently set photographing conditions according to conditions such as sensed welding light at the welding site, ambient light, and the degree of movement of the welding torch 200.

Specifically, the first processor 150 may set photographing conditions so that, for example, as welding light and/or ambient light at a welding site increases, at least one of ISO sensitivity, gain, and shutter speed decreases.

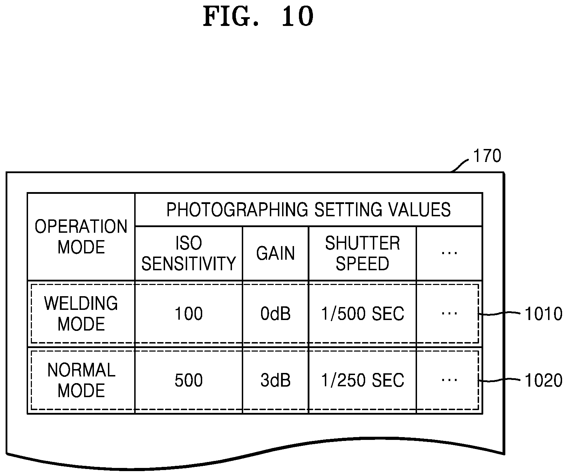

In an embodiment, the first processor 150 (or the controller 114 of FIG. 4) may operate the camera unit 110 (specifically, the camera module 112 in the camera unit 110) according to the intensity of light in the welding operation area, in any one of a plurality of operation modes with different photographing conditions. Here, the first processor 150 may change the operation mode of the camera unit 110 into a first mode (for example, 'welding mode') and a second mode (for example, 'normal mode') based on the intensity of light detected by the illuminance sensor 141. By changing the photographing conditions of the camera unit 110 to photographing setting values preset in the memory 170 in response to the determined mode, the first processor 150 may quickly switch the photographing conditions of the camera unit 110 without delay. Here, the memory 170 may store photographing setting values for each mode (see FIG. 10).

When the intensity of light is greater than or equal to a preset threshold value, the first processor 150 may determine the operation mode of the camera unit 110 to be a first mode (e.g., 'welding mode'), and may change the photographing conditions of the camera unit 110 to photographing setting values (1010 of FIG. 10) detected from the memory 170 according to the first mode. In addition, when the intensity of light is less than the preset threshold value, the first processor 150 may determine the operation mode of the camera unit 110 to be a second mode (e.g., 'normal mode'), and may change the photographing conditions of the camera unit 110 to photographing setting values (1020 of FIG. 10) detected from the memory 170 according to the first mode.

In an embodiment, the first processor 150 (or the controller 114 of FIG. 4) may control the operation mode of the camera unit 110 based on a detection result of the illuminance sensor 141 that detects light at intervals of a set cycle or continuously. The first processor 150 may count the number of times the operation mode of the camera unit 110 (camera module) (or the photographing conditions of the camera unit) changes according to a change in light intensity, and when the counted number of times exceeds a preset number during a preset period of time, the first processor 150 may generate a notification message and output the notification message, for example, on the display unit 130, thereby limiting the progress of frequent welding operation within a short period of time to protect the eyes.

In another embodiment, the first processor 150 may determine the operation mode of the camera unit 110 based on the intensity of light in the welding operation area, detected by the illuminance sensor 141, but is not limited thereto.

The first processor 150 may estimate the intensity of light within the welding operation area by using the image sensor 112-4 and determine the operation mode of the camera unit 110 based on the estimated intensity of light. Here, the first processor 150 may estimate the intensity of light in the welding operation area based on an image signal generated by recognizing light in the welding operation by the image sensor 112-4, or may estimate the intensity of light in the welding operation area based on a welding image frame obtained by the camera unit 110 based on the image signal.

For example, the first processor 150 may first determine the operation mode of the camera unit 110 based on the intensity of light in the welding operation area, which is estimated using the image sensor 112-4, and when the change in the intensity of light (or a change in the operation mode of the camera unit) is a set condition, the first processor 150 may determine the operation mode of the camera unit 110 based on the intensity of light detected by the illuminance sensor 141. The set condition may be, for example, a case where the change in the intensity of light estimated using the image sensor 112-4 during a set time is less than a set value (or when the operation mode of the camera unit 110 does not change during the set time). That is, when a change in the intensity of light is not properly detected using the image sensor 112-4, the first processor 150 may detect the intensity of light more accurately through the illuminance sensor 141, thereby sensitively controlling the operation mode of the camera unit 110 according to the surrounding environment.

In an embodiment, by using a deep learning-based light estimation learning model that is previously trained to estimate the intensity of light from a signal about an image (or a frame about an image), the first processor 150 may estimate the intensity of light from an image signal generated by the image sensor 112-4 (or the welding image frame obtained by the camera unit 110). For example, the light estimation learning model may be trained using supervised learning using training data that uses a signal about an image (or a frame about an image) as an input and the intensity of light as a label, but it not limited thereto.

The first processor 150 may determine the operation mode of the camera unit 110 by using the illuminance sensor 141 after determining the operation mode of the camera unit 110 by using the image sensor 112-4, but is limited thereto. Alternatively, the first processor 150 may determine the operation mode of the camera unit 110 by using the image sensor 112-4 after determining the operation mode of the camera unit 110 by using the illuminance sensor 141. In an embodiment, the first processor 150 may determine the operation mode of the camera unit 110 by using at least one of the illuminance sensor 141 and the image sensor 112-4. Additionally, the first processor 150 may determine the operation mode of the camera unit 110 by using the image sensor 112-4 and the illuminance sensor 141 according to a set priority.

Also, when it is sensed that the movement and/or the working speed of the welding torch (200 of FIG. 1) are fast, the photographing condition may be set to increase the shutter speed.

The first processor 150 may synthesize images having a preset number of frames in parallel. According to an embodiment of the present disclosure, each image in the preset frame may be captured under different photographing conditions.

When there are two or more camera units 110, the first processor 150 according to an embodiment of the present disclosure may control each camera unit to capture images under different photographing conditions. Even in this case, the first processor 150 may synthesize images having a preset number of frames in parallel.

The first processor 150 may be located outside the camera unit 110 and may control the camera unit 110, but is not limited thereto. For example, as shown in FIG. 4, the controller 114 that controls the camera unit 110 may be located inside the camera unit 110, and the controller 114 may communicate with the first processor 150. The controller 114 may receive photographing setting values according to the determined operation mode of the camera unit 110 from the first processor 150 and change the photographing conditions of the camera unit 110 to the received photographing setting values. However, the present disclosure is not limited thereto and may perform all functions of the first processor 150 related to the camera unit 110. For example, the controller 114 of the camera unit 110 may determine the operation mode of the camera unit 110, and change the photographing conditions of the camera unit 110 to preset photographing setting values corresponding to the operation mode of the camera unit 110. Here, the photographing setting values may be stored in the memory 170 or in a memory (not shown) within the camera unit 110. Additionally, the controller 114 may be configured to include the second processor 113-5.

The first processor 150 may control the overall operation of the welding information providing apparatus 100 by using various types of programs stored in the memory 170. For example, the first processor 150 may include a central processing unit (CPU), random access memory (RAM), read-only memory (ROM), and a system bus. Here, the ROM is an element in which a set of instructions for system booting are stored, and the CPU copies an operating system (O/S) stored in the memory of the welding information providing apparatus 100 to the RAM according to the instructions stored in the ROM and executes the O/S to boot the system. When booting is finished, the CPU may copy various applications stored in the memory to the RAM and execute the applications to perform various operations. The first processor 150 has been described above as including only one CPU, but may be implemented with a plurality of CPUs (or digital signal processors (DSPs), system on chips (SoCs), or the like).

According to an embodiment of the present disclosure, the first processor 150 may be implemented as a digital signal processor (DSP) for processing a digital signal, a microprocessor, and/or a time controller (TCON). However, one or more embodiments are not limited thereto, and the processor may include one or more from a CPU, a micro controller unit (MCU), a micro processing unit (MPU), a controller, an application processor (AP), a communication processor (CP), and an advanced RISC machine (ARM) processor, or may be defined by a corresponding term. In addition, the first processor 150 may be implemented as a system on chip (SoC) having a processing algorithm provided therein, or a large-scale integration (LSI), or may be implemented in the form of a field programmable gate array (FPGA).

Although not shown in FIG. 2, the welding torch 200 may include a communication unit, a sensor unit, and a third processor.

The communication unit of the welding torch 200 transmits and receives data to and from the welding information providing apparatus 100. The communication unit may include a module capable of short-range wireless communication (e.g., Bluetooth, Wifi, Wifi-Direct) or long-distance wireless communication (3G, High-Speed Downlink Packet Access (HSDPA) or LTE).

The sensor unit or second sensor of the welding torch 200 is included in the welding torch (200 in FIG. 1) and may be configured to sense the welding situation, such as welding temperature, welding speed, welding slope, welding direction, and a gap between a base material and the welding torch.

The sensor unit may detect at least one of various changes such as a change in posture of a user holding the welding torch 200, a change in illuminance of the welding surface, a change in an acceleration of the welding torch 200, etc., and may transmit an electrical signal corresponding to the change to the third processor. That is, the sensor unit may sense a state change made based on the welding torch 200, generate a sensing signal corresponding thereto, and transmit the sensing signal to the third processor.

In the present disclosure, the sensor unit may include various sensors, and when the welding torch 200 is driven (or based on a user setting), power is supplied to at least one sensor that is preset according to control to sense a change in the status of the welding torch 200.

In this case, the sensor unit may be configured to include at least one of all types of sensing devices capable of detecting a change in the status of the welding torch 200. For example, the sensor unit may include at least one of various sensing devices such as an acceleration sensor, a gyro sensor, an illuminance sensor, a proximity sensor, a pressure sensor, a noise sensor, a video sensor, a gravity sensor, etc. A degree of light within a welding operation area, which is detected via the illuminance sensor of the welding torch 200, may be transmitted to the first processor 150 through the communication unit 120, and the first processor 150 may control the lighting unit 160 and/or the camera unit 110 on the basis of the degree of light transmitted via the illuminance sensor of the welding torch 200 rather than the sensor unit 140 of the welding information providing apparatus 100.

Meanwhile, the acceleration sensor is a component for sensing movement of the welding torch 200. In detail, the acceleration sensor may measure dynamic forces such as acceleration, vibration, and impact of the welding torch 200, and thus may measure the movement of the welding torch 200.

The gravity sensor is an element for sensing a direction of gravity. That is, a sensing result of the gravity sensor may be used to determine the movement of the welding torch 200 along with the acceleration sensor. In addition, a direction in which the welding torch 200 is gripped may be determined via the gravity sensor.

In addition to the above-described types of sensors, the welding torch 200 may further include various types of sensors such as a gyroscope sensor, a geomagnetic sensor, an ultrasonic sensor, and a radio frequency (RF) sensor, and may detect various changes regarding the welding operation environment.

FIGS. 5 to 8 are diagrams for describing the welding information providing apparatus 100 according to an embodiment of the present disclosure.

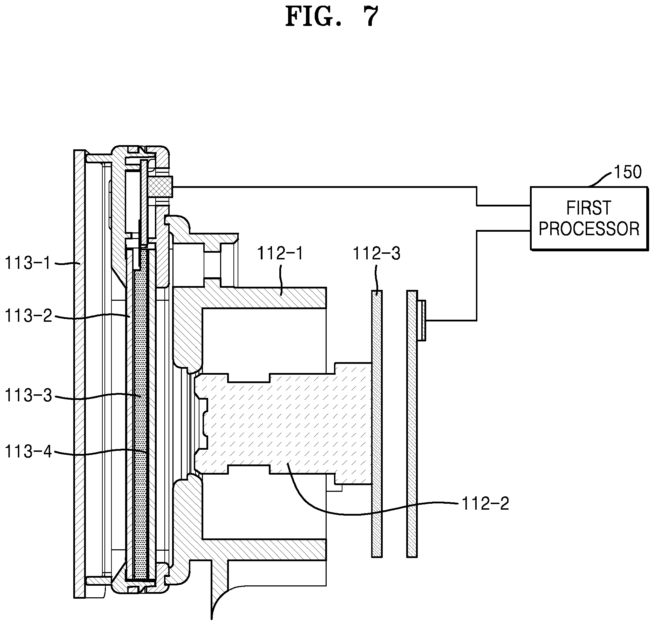

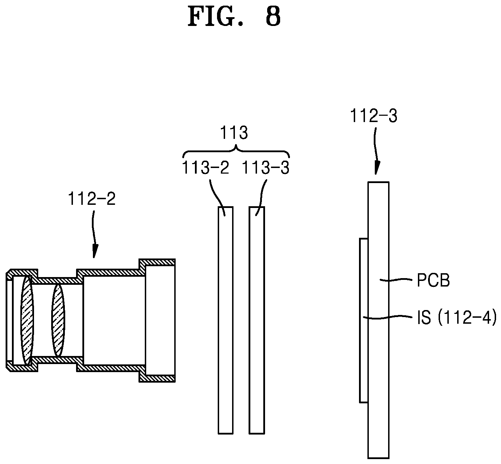

FIG. 5 is a perspective view of the welding information providing apparatus 100 according to an embodiment of the present disclosure, and FIG. 6 is a cross-sectional view for describing inside of the welding information providing apparatus 100 of FIG. 5. FIG. 7 is a diagram illustrating only the configuration of the camera unit 110 of FIG. 6, and FIG. 8 is a diagram of the camera unit 110 according to another embodiment.

Referring to FIGS. 5 and 6, the welding information providing apparatus 100 of the present disclosure may include the main body 101, the display unit 130 installed on the front surface of the main body 101, the camera unit 110, and the first processor 150. In addition, the welding information providing apparatus 100 may include at least one sensor unit (see 140, FIG. 2) and a fixing unit (not shown) that is arranged on a rear surface of the main body 101 to fix the welding information providing apparatus 100 on the head of the operator.

According to an embodiment, the camera unit 110 may be mounted on the outside of the main body 101 and may be implemented as one or more. When there is one camera unit 110, the camera unit 110 may be mounted on the upper end at the center of the main body 101. In another embodiment, when there are two camera units 110, the camera units 110 may be symmetrically mounted on one area of the front portion of the display unit 130. Here, the front portion of the display unit 130 may be an external area (the area as shown in FIG. 5) corresponding to the direction in which the welding operation is performed. In contrast, a rear portion of the display unit 130 may be an internal region corresponding to a direction of the face of the operator. However, the present disclosure is not limited thereto, and the camera unit 110 may be arranged on an external area of the main body 101, the external area corresponding to the direction in which the welding operation is performed. Also, the camera unit 110 may be formed to be attachable/detachable so as to change its position as necessary.

In addition, the welding information providing apparatus 100 of the present disclosure may include a main lens member 135 for transferring a welding image provided by the display 131 to both eyes of the user at a short distance.

The welding information providing apparatus 100 according to an embodiment of the present disclosure provides the welding image via the display 131 that is spaced a certain distance apart from the user in order to ensure a viewing area of the user. As such, the user may be provided with the welding image from one display, instead of the displays corresponding respectively to both eyes, and thus, a wide field of view may be secured even in a sealed space in the welding information providing apparatus 100. However, in this case, the welding information providing apparatus 100 may include the main lens member 135 in order to clearly provide the user with the welding image provided from the display 131.

When there is one display 131 according to the present disclosure, a virtual center line passing a center of the display unit 130 may be located between both eyes of the user. However, the present disclosure is not limited thereto, and a plurality of displays 131 may be also arranged at positions that are spaced certain distances from both eyes of the user.

The main lens member 135 is arranged on a path through which the welding image provided from the display 131 passes, and may have a convex surface so as to adjust a size of the welding image and guide the welding image to both eyes of the user. The main lens member 135 may be a convex lens including a convex surface for enlarging the welding image.

The main lens member 135 may include a glass material, a plastic material, etc. When the main lens member 135 includes a plastic material, a styrene resin including (meth)acrylic resin, allyl carbonate resin such as polycarbonate resin, allyl resin, diethylene glycol bisallyl carbonate resin (CR-39), etc., vinyl resin, polyester resin, polyether resin, urethane resin obtained by reaction of an isocyanate compound with a hydroxy compound such as diethylene glycol, etc., thiourethane resin obtained by reacting an isocyanate compound with a polythiol compound, a transparent resin obtained by curing a polymerizable composition containing a (thio)epoxy compound having at least one disulfide bond in the molecule, etc. may be used. In addition, the main lens member 135 may be coated with a filter that blocks light of a certain wavelength band. For example, the main lens member 135 may be a lens coated with a blue light blocking filter.

The main lens member 135 may be moved back and forth based on an initial position. As described above, the power of the main lens member 135 is determined in consideration of the user's eyesight, but because the eyesight is different for each user, the welding information providing apparatus 100 of the present disclosure may provide the welding image having the best image quality by adjusting the distance between the main lens member 135 and the user. To this end, the welding information providing apparatus 100 of the present disclosure may further include a distance adjusting member (not shown) for adjusting the distance between the main lens member 135 and the display 131.

As with the main lens member 135, the display unit 130 may further include a distance adjusting member to adjust a distance to the main lens member 135 or to adjust a distance between the display 131 and the user's eyes. For example, the welding information providing apparatus 100 may further include a barrel support for fixing the display unit 130 to one side. The distance adjusting member may move the barrel support back and forth according to manual distance adjustment by the user or automatic distance adjustment. As such, the display unit 130 may be depressed inside the front portion of the main body 101 or may protrude to outside of the front portion.

At least one sensor (140 or a first sensor) may be mounted on one area of the front portion of the display unit 130. In another embodiment, the sensor unit 140 may be included in the main body 101. Here, the sensor unit 140 may be mounted in the front direction of the main body 101 to sense the welding situation.

The main body 101 protecting the face of the operator may include a material having a certain strength, for example, reinforced plastic, but the present disclosure is not limited thereto, that is, may include various materials having resistance against such elements as sparks that may generate during a welding operation.

The fixing unit (not shown) is an element that comes into direct contact with the head of the operation, and thus, a side surface of the fixing unit (not shown), that is, at least a part of an internal surface coming into direct contact with the head of the operator, may include a soft material such as fiber or cushion material.

Hereinafter, the camera unit 110 will be described in more detail with reference to the drawings.

Referring again to FIGS. 6 and 7, the camera unit 110 according to an embodiment of the present disclosure may include an exterior housing 111, a camera module 112 provided inside the exterior housing 111, and a light blocking panel 113. The camera unit 110 may further include at least one of an illuminance sensor that detects the intensity of light in the welding operation area, a lighting unit that irradiates illumination light, and a controller that generally controls the operation of the camera unit 110.

The camera module 112 may include a camera housing 112-1, a lens assembly 112-2, and a substrate assembly 112-3, and obtain a welding image frame in a welding operation area according to photographing conditions (e.g., shutter speed, ISO sensitivity, gain, etc.).

The camera housing 121 performs a function of fixing the camera module 112 including the lens assembly 112-2 and the substrate assembly 112-3. The camera housing 112-1 has a through hole in a region corresponding to the lens assembly 112-2 so that welding light enters the camera module 112.

The lens assembly 112-2 may include an actuator and one or more lenses accommodated therein. The actuator may include an actuator for auto-focusing and/or an actuator for stabilization, and the actuator for auto-focusing and the actuator for stabilization may be integrally provided.

The substrate assembly 112-3 may have a structure in which an image sensor IS (112-4 in FIG. 8) is coupled to a printed circuit board (PCB) on which a conductive wiring pattern is formed.

The light blocking panel 113 may function to block welding light (or ambient light) generated by a welding operation. The camera unit 110 may capture a welding image frame in which a certain amount of welding light is shielded using the light blocking panel 113, through the camera module 112. The light blocking panel 113 may be arranged in front of the camera, in detail, may be located in front of the lens receiving light from a subject.

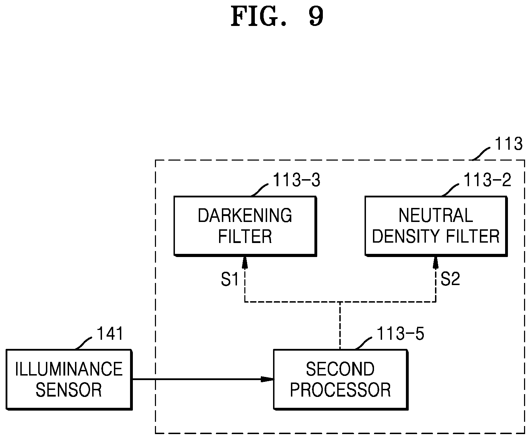

As an example, the light blocking panel 113 may include a darkening filter 113-3 and a second processor (113-5 in FIG. 9). The darkening filter 113-3 may block the welding light generated when the operator performs the welding operation. That is, the second processor 113-5 may darken the darkening filter 113-3 based on the welding light information (or ambient light information) detected through the illuminance sensor 141 (or optical sensor) of the sensor unit 140, thereby increasing the light blocking degree of the darkening filter 113-3. Here, the darkening filter 113-3 may include, for example, a liquid crystal display panel in which the darkening concentration may be adjusted according to an alignment direction of the liquid crystal. However, the present disclosure is not limited thereto, and the darkening filter 113-3 may be implemented with various panels such as vertical align (VA) type LCD, twist nematic (TN) type LCD, in plane switching (IPS) type LCD, etc.

The darkening density of the darkening filter 113-3 may be automatically adjusted by the second processor 113-5 depending on the presence or absence of welding light or the intensity of welding light (or ambient light). As described above, when the brightness of the welding light is automatically adjusted, the illuminance sensor 141 of the sensor unit 140 may be used. The illuminance sensor 141 of the sensor unit 140 may obtain welding light information by sensing whether there is the welding light or the intensity of the welding light, and may convert information about the intensity of the welding light included in the welding light information into a certain electrical signal. The illuminance sensor 141 of the sensor unit 140 may transmit the converted electrical signal to the second processor 113-5, and the second processor 113-5 may control the darkening density based on the intensity of the welding light. The darkening filter 113-3 may be controlled by the second processor 113-5, but is not limited thereto and may also be controlled by the first processor 150.

That is, the darkening filter 113-3 may change the light blocking degree of the panel in real-time to correspond to the intensity of light generated from the welding surface at the welding operation site, and the camera unit 110 may capture, by using the camera module 112, a welding image frame in which a certain amount of welding light is shielded by the darkening filter 113-3 installed on the front portion thereof.

In another embodiment, the light blocking panel 113 may further include a neutral density filter 113-2. The neutral density filter 113-2 (e.g., variable neutral density filter) may transmit welding light generated when the operator performs the welding operation, to the camera module 112, after reducing the intensity of the welding light. The neutral density filter 113-2 may reduce the intensity of the welding light according to a neutral density set in advance. For example, when the neutral density filter 113-2 having the neutral density of 50% is applied, the intensity of the welding light may be further reduced as compared with the case in which the neutral density filter 113-2 having the neutral density of 15% is applied.

The light blocking panel 113 may have a structure in which the neutral density filter 113-2 is replaceable. The operator may use the neutral density filter 113-2 of a different neutral density after replacing as necessary.

As another embodiment, the neutral density filter 113-2 may be a digital filter, the neutral density of which is controlled by the second processor 113-5 (or the first processor 150). As with the darkening filter 113-3, the neutral density filter 113-2, that is, a digital filter, may increase the neutral density based on the welding light information (or ambient light) sensed by the sensor unit 140, e.g., the illuminance sensor 141, and increase the light blocking degree of the neutral density filter 113-2.

Here, the neutral density filter 113-2 may be located in front of the camera module 112 to be farther from the camera module 112 than the darkening filter 113-3. Because the darkening filter 113-3 has a greater degree of blocking the welding light as compared with the neutral density filter 113-2, the camera unit 110 may block the light by using the neutral density filter 113-2 according to the intensity of the welding light, and then, when the intensity of the welding light is large, the camera unit 110 may block the light by using the darkening filter 113-3 or by using both the darkening filter 113-3 and the neutral density filter 113-2.

Although not shown in the drawings, the light blocking panel 113 may further include a bandpass filter. The neutral density filter 113-2 or the darkening filter 113-3 performs a function of reducing the intensity of the welding light regardless of the wavelength of the light, but the bandpass filter may perform a function of blocking light of a certain wavelength band. When the light blocking panel 113 further includes the bandpass filter (not shown), the light blocking panel 113 may block ultraviolet ray or infrared ray generated during the welding operation. A bandpass filter (not shown) blocks the infrared and ultraviolet rays, and may be disposed to be the farthest from the face of the operator. The bandpass filter (not shown) may have a dielectric multilayer formed on a surface facing the other filters.

When the welding light is generated during the welding operation of the operator, the infrared ray and the ultraviolet ray in the welding light are reflected and blocked by the dielectric multilayer of the bandpass filter, and visible light only passes through the bandpass filter and may be irradiated toward the camera module 112.

The light blocking panel 113 may have a protective glass 113-1 on a frontmost portion thereof facing the welding light, in order to deal with an accident such as splashing of the spark generated during the welding operation. A coating layer that may prevent fogging or moisture generation by using a hydrophilic coating material may be formed on a front portion of the protective glass 113-1. As such, the light blocking panel 113 may prevent contaminants from attaching to the light blocking panel 113.

In detail, the coating layer formed on the front surface of the protective glass 113-1 may include a hydrophilic photocatalytic material such as titanium dioxide (TiO₂) having a self-cleaning performance, in order to prevent adsorption of organic materials around the welding operation.

In addition, the light blocking panel 113 may further include a glass portion 113-4 disposed between the darkening filter 113-3 and the face of the operator. The glass portion 113-4 may be directly or indirectly coupled to the darkening filter 113-3 so as to prevent the darkening filter 113-3 from being damaged due to external foreign matters. The glass portion 113-4 may include a transparent material.

In another embodiment, referring to FIG. 8, the light blocking panel 113 may be coupled to the camera module. The light blocking panel 113 may be disposed on a path through which light moves inside the lens assembly 112-2 of the camera module 112. The light blocking panel 113 may be disposed on the front portion of the lens assembly 112-2 that receives light, or may be disposed between the lens assembly 112-2 and the image sensor IS 112-4 to block light. In other words, the light blocking panel 113 forms the exterior by using an additional housing, but may be formed to be coupled to the camera module 112. The light blocking panel 113 may be coupled to the front portion of the camera module 112 or may be disposed between the lens assembly 112-2 of the camera module 112 and the image sensor IS 112-4 and coupled therebetween. In addition, in another embodiment, the light blocking panel 113 may be integrally formed with the lens assembly 112-2 of the camera module 112. The darkening filter 113-3 and the variable neutral density filter 113-2 of the light blocking panel 113 are arranged on an optical axis of a lens and may be coupled via one housing. In this case, the light blocking panel 113 may be formed in a circular shape that is similar to the shape of the lens L. However, the present disclosure is not limited thereto, and the light blocking panel 113 may be formed in a different shape from the lens L, for example, a rectangular shape. In this case, the lens assembly 112-2 may have a housing with an internal structure capable of accommodating both the circular lens L and the rectangular light blocking panel 113.

In this case, as described above, the light blocking panel 113 may include the darkening filter 113-3 and may further include the neutral density filter 113-2 that is a digital neutral density filter. Also, although not shown in the drawings, the light blocking panel 113 may further include a bandpass filter.

Hereinafter, a method of controlling the light blocking panel 113 by the second processor 113-5 will be described. FIG. 9 is a block diagram for describing a method of controlling a light blocking panel within a camera unit.

Referring to FIG. 9, the welding information providing apparatus 100 according to an embodiment of the present disclosure may obtain welding light information by the sensor unit 140. Here, the sensor unit 140 is an optical sensor and includes the illuminance sensor 141, and may detect the presence or intensity of welding light. The illuminance sensor 141 may convert the presence or intensity of welding light into an electrical signal and transmit the electrical signal to the second processor 113-5 of the light blocking panel 113. The light blocking panel 113 of the camera unit 110 may include, along with the second processor 113-5, the darkening filter 113-3 and the neutral density filter 113-2 that are controllable. When an electrical signal is received from the illuminance sensor 141, the second processor 113-5 may generate a control signal and directly transmit the control signal to the darkening filter 113-3 and the neutral density filter 113-2, thereby immediately controlling the degree of light blocking of the light blocking panel 113 according to a light detection result of the illuminance sensor 141.

When the welding light detected through the illuminance sensor 141 exceeds a preset first intensity, the second processor 113-5 may generate a first control signal S1 and a second control signal S2 and control both the darkening filter 113-3 and the neutral density filter 113-2 to operate, thereby controlling the light blocking degree of the light blocking panel 113. The second processor 113-5 may adjust both the darkening density of the darkening filter 113-3 and the neutral density of the neutral density filter 113-2 according to the intensity of the detected welding light.

When the welding light detected through the illuminance sensor 141 is equal to or less than the preset first intensity but exceeds a set second intensity, the second processor 113-5 may generate the second control signal S2 to control only the neutral density filter 113-2, or generate the first control signal S1 to control only the darkening filter 113-3, thereby controlling the light blocking degree of the light blocking panel 113.

In addition, when no welding light is sensed through the illuminance sensor 141 or welding light is equal to or less than the second intensity set in advance, the second processor 113-5 may not separately control the neutral density filter 113-2 or the darkening filter 113-3, but transfer the welding light as it is to the camera module 112.

In an embodiment, the second processor 113-5 may generate the first control signal S1 and the second control signal S2 to control the light blocking degree of the light blocking panel 113 in proportion to the intensity of light sensed by the illuminance sensor141, or may generate the first control signal S1 and the second control signal S2 such that the light blocking degree of the light blocking panel 113 becomes a preset value based on a result of comparison between the intensity of light and a preset intensity.

A welding information providing apparatus according to one or more embodiments of the present disclosure may obtain a welding image (frame), in which a certain amount of welding light is shielded, without separately processing a captured image, by arranging, in front of a camera, a light blocking panel that may shield the welding light. Also, the welding information providing apparatus may effectively control a shielding degree by including a darkening filter and a neutral density filter and controlling a darkening density or a neutral density according to whether there is welding light or an intensity of the welding light.

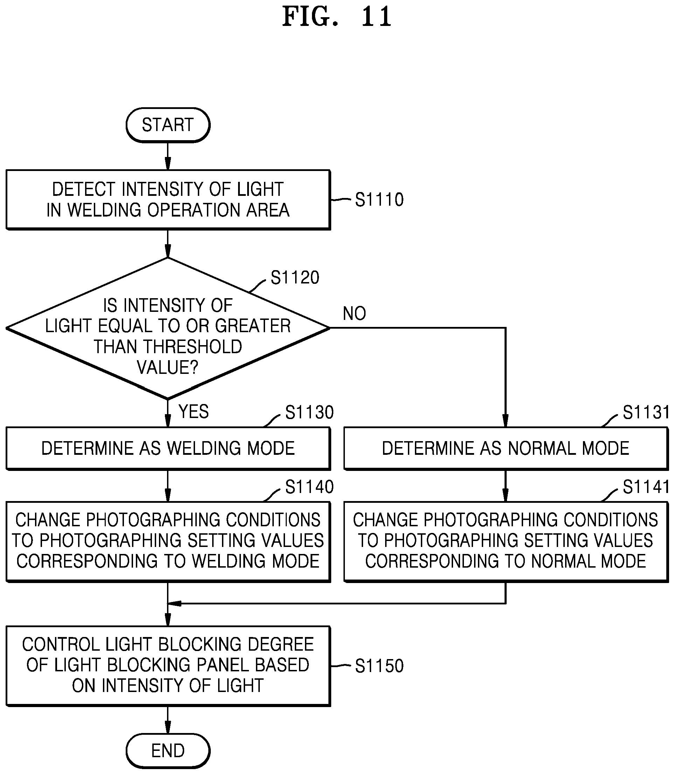

FIG. 11 is a flowchart for describing an operation method of a welding information providing apparatus, according to this embodiment. The operation method of the welding information providing apparatus (or a control method for a camera unit) may be performed by the welding information providing apparatus (or camera unit) according to the present embodiment.

Referring to FIG. 11, in operation S1110, the welding information providing apparatus may detect intensity of light in a welding operation area through an illuminance sensor.

In operation S1120, if the intensity of light detected by the illuminance sensor is equal to or greater than a preset threshold value, in operation S1130, through a first processor, the welding information providing apparatus may determine the operation mode of the camera unit to be a first mode (e.g., 'welding mode').

In operation S1140, the welding information providing apparatus may change the photographing conditions of the camera unit to the photographing setting values (1010 in FIG. 10) detected from the memory in accordance with the first mode. Here, the photographing setting values corresponding to the first mode may be ISO sensitivity, gain, shutter speed, etc. corresponding to the 'welding mode'.

In operation S1120, when the intensity of light detected by the illuminance sensor is less than the preset threshold value, in operation S1131, the welding information providing apparatus may determine the operation mode of the camera unit to be a second mode (e.g., 'normal mode'), and may change in operation S1141, the photographing conditions of the camera unit to the photographing setting values (1020 in FIG. 10) detected from the memory in accordance with the second mode. The photographing setting values corresponding to the second mode may be ISO sensitivity, gain, shutter speed, etc. corresponding to the 'normal mode'.

Here, the ISO sensitivity, gain, and shutter speed corresponding to the 'welding mode' may be respectively smaller than the ISO sensitivity, gain, and shutter speed corresponding to the 'normal mode'.

The first processor of the welding information providing apparatus may determine 'welding mode' or 'normal mode' as an operation mode of the camera unit based on the intensity of light detected by the illuminance sensor, and may quickly change the photographing conditions of the camera unit to photographing setting values corresponding to the determined mode.

In operation S1150, the welding information providing apparatus may control the light blocking degree of a light blocking panel based on the intensity of light. The welding information providing apparatus may control the light blocking degree of the light blocking panel based on the intensity of light through a second processor of the light blocking panel, thereby immediately controlling the light blocking degree of the light blocking panel according to a light detection result. Here, the welding information providing apparatus may control the light blocking degree of the light blocking panel in proportion to the intensity of light, or may control the light blocking degree of the light blocking panel to be a preset value based on a comparison result between the light intensity and the preset intensity. Additionally, in an embodiment, the welding information providing apparatus may change the light blocking degree of the light blocking panel to a preset value in response to the operation mode of the camera unit.

As described above, the welding information providing apparatus according to the embodiments of the present disclosure adaptively changes the operation mode of the camera unit and photographing conditions according to the operation mode to obtain and provide a clear image of a welding operation, thereby allowing operators to accurately recognize the welding process.

The welding information providing apparatus according to the embodiments of the present disclosure may determine the operation mode of the camera unit to be one of the first mode and the second mode according to the intensity of light in the welding operation area, detected by the illuminance sensor, and change the photographing conditions of the camera unit to the photographing setting values preset in the memory according to the determined mode, thereby quickly changing the photographing conditions of the camera unit without delay.

The welding information providing apparatus according to the embodiments of the present disclosure may immediately control, through a processor separately provided in the light blocking panel, the light blocking degree of the light blocking panel according to the intensity of light in the welding operation area, detected by the illuminance sensor, and obtain a welding image in which a certain amount of welding light is shielded, without separately processing the captured image.

The embodiments according to the present disclosure described above may be implemented in the form of a computer program that can be executed through various components on a computer, and such a computer program may be recorded on a computer-readable medium. The media may include magnetic media such as hard disks, floppy disks, and magnetic tapes, optical recording media such as CD-ROMs and DVDs, magneto-optical media such as floptical disks, and hardware devices specifically configured to store and execute program instructions such as ROM, RAM, flash memory, etc.

Meanwhile, the computer program may be designed and configured specifically for the present disclosure, or may be well-known and available to one of ordinary skill in the art of computer software. Examples of the computer program may include advanced language codes that can be executed by a computer by using an interpreter or the like as well as machine language codes made by a compiler.

The use of the terms "a" and "an" and "the" and similar referents in the context of describing the present disclosure (especially in the context of the following claims) are to be construed to cover both the singular and the plural. Furthermore, recitation of ranges of values herein are merely intended to serve as a shorthand method of referring individually to each separate value falling within the range, unless otherwise indicated herein, and each separate value is incorporated into the specification as if it were individually recited herein.

The steps of all methods described herein can be performed in any suitable order unless otherwise indicated herein or otherwise clearly contradicted by context. The present disclosure is not necessarily limited by the order of description of the above steps. The use of any and all examples, or exemplary language (e.g., "such as") provided herein, is intended merely to better illuminate the present disclosure and does not pose a limitation on the scope of the present disclosure unless otherwise claimed. Numerous modifications and adaptations will be readily apparent to those skilled in this art without departing from the spirit and scope of the present disclosure.

Therefore, the spirit of the present disclosure should not be limited to the embodiments described above, and the scope of the claims described below as well as all scopes equivalent to or equivalently changed from the scope of the claims are within the scope of the spirit of the present disclosure.

According to an embodiment of the present disclosure, a camera unit and a welding information providing apparatus including the camera unit may be provided, wherein the operation mode of a camera module and photographing conditions according to the operation mode may be adaptively changed to obtain and provide a clear image of a welding operation so as to allow operators to accurately recognize the welding process.

According to an embodiment of the present disclosure, the operation mode of the camera unit may be determined to be one of the first mode and the second mode according to the intensity of light in the welding operation area, detected by the illuminance sensor, and the photographing conditions of the camera unit may be changed to the photographing setting values preset in the memory according to the determined mode, thereby quickly changing the photographing conditions of the camera unit without delay.

In addition, according to an embodiment of the present disclosure, the light blocking degree of the light blocking panel may be immediately controlled, through a processor separately provided in the light blocking panel, according to the intensity of light in the welding operation area, detected by the illuminance sensor, so as to obtain a welding image in which a certain amount of welding light is shielded, without separately processing the captured image.

The effects of the present disclosure are not limited to those mentioned above, and other effects not mentioned will be clearly understood by those skilled in the art from the description below.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A camera unit comprising:
a camera module configured to obtain a welding image frame in a welding operation area according to photographing conditions;
an illuminance sensor configured to detect an intensity of light within the welding operation area; and
a controller configured to determine, based on the intensity of light detected by the illuminance sensor, an operation mode of the camera module to be one of a first mode and a second mode, and change photographing conditions of the camera module to preset photographing setting values according to the determined mode.

2. The camera unit of claim 1, wherein the camera module comprises an image sensor that recognizes light from a welding operation, and
the camera unit further comprises a light blocking panel disposed in front of the camera module including the image sensor and configured to block a portion of light incident on the welding operation and transmit the remaining light to the image sensor.

3. The camera unit of claim 2, wherein the light blocking panel comprises a processor electrically connected to the illuminance sensor, and
the processor is configured to control a light blocking degree of the light blocking panel based on the intensity of the light.

4. The camera unit of claim 3, wherein the processor is further configured to control the light blocking degree of the light blocking panel in proportion to the intensity of the light, or control the light blocking degree of the light blocking panel to be a preset value based on a comparison result between the intensity of the light and a preset intensity.

5. The camera unit of any of the claims 1 to 4, further comprising a lighting unit that irradiates illumination light,
wherein the controller adjusts an output intensity of the illumination light emitted from the lighting unit, based on the intensity of the light.

6. The camera unit of any of the claims 1 to 5, wherein the illuminance sensor detects the intensity of the light at intervals of a set cycle, and
the controller counts the number of times the operation mode of the camera module changes according to a change in the intensity of the light, and generates and outputs a notification message when the counted number exceeds a preset number during a preset time.

7. A welding information providing apparatus comprising:
a main body provided to be worn by a user;
a camera unit disposed outside the main body and configured to obtain a welding image frame in a welding operation area according to photographing conditions;
an illuminance sensor configured to detect an intensity of light within the welding operation area; and
a first processor configured to determine, based on an intensity of light detected by the illuminance sensor, an operation mode of the camera unit to be one of a first mode and a second mode, and change photographing conditions of the camera unit to preset photographing setting values according to the determined mode.

8. The welding information providing apparatus of claim 7, wherein the camera unit comprises an image sensor that recognizes light from a welding operation, and a light blocking panel disposed in front of the image sensor and configured to block a portion of light incident on the welding operation and transmit the remaining light to the image sensor.

9. The welding information providing apparatus of claim 8, wherein the light blocking panel comprises a second processor electrically connected to the illuminance sensor, and
the second processor is configured to control a light blocking degree of the light blocking panel based on the intensity of the light.

10. The welding information providing apparatus of claim 9, wherein the second processor is further configured to control the light blocking degree of the light blocking panel in proportion to the intensity of the light, or control the light blocking degree of the light blocking panel to be a preset value based on a comparison result between the intensity of the light and a preset intensity.

11. The welding information providing apparatus of any of the claims 7 to 10, further comprising a lighting unit that irradiates illumination light,
wherein the first processor adjusts an output intensity of the illumination light emitted from the lighting unit, based on the intensity of the light.

12. The welding information providing apparatus of any of the claims 7 to 11, wherein the illuminance sensor detects the intensity of the light at intervals of a set cycle, and
the first processor counts the number of times the operation mode of the camera module changes according to a change in the intensity of the light, and generates and outputs a notification message when the counted number exceeds a preset number during a preset time.
